# EUROPÄISCHE PATENTSCHRIFT

(11) **EP 0 915 686 B1**
(45) Veröffentlichungstag und Bekanntmachung des Hinweises auf die Patenterteilung: **31.07.2002**
(21) Anmeldenummer: 97923008.3
(22) Anmeldetag: 07.05.1997
(51) Int. Cl.: A61F 2/30

(54) **ENDOPROTHESE**
ENDOPROSTHESIS
ENDOPROTHESE

(30) Priorität: 24.07.1996 DE 29612857 U
(43) Veröffentlichungstag der Anmeldung: 19.05.1999
(73) Patentinhaber: Waldemar Link (GmbH & Co.), 22339 Hamburg (DE)
(72) Erfinder: KELLER, Arnold, D-23863 Kayhude (DE)
(74) Vertreter: Glawe, Delfs, Moll & Partner
(86) Internationale Anmeldenummer: EP9702344
(87) Internationale Veröffentlichungsnummer: WO9804215

(56) Entgegenhaltungen:
- EP-A- 0 474 015
- US-A- 5 336 268

## Beschreibung

Es sind Endoprothesen bekannt (EP-A-474 015), deren Komponenten über eine Konusverbindung miteinander verbunden sind. Diese umfaßt eine Konusbohrung an einer Komponente und einen dazu passenden, von der Konusbohrung aufgenommenen Konuszapfen an der anderen Komponente. Bei Wahl eines hinreichend geringen Konuswinkels sitzen diese fest ineinander, sofern sie bei der Montage mit hinreichender Kraft zusammengefügt wurden. Um gegen Zufälligkeiten gesichert zu sein, ist diese Konusverbindung mit einer Sicherungsschraube versehen, die in einer Gewindebohrung der einen Komponente sitzt und quer zur Löserichtung mit ihrer Spitze in eine entsprechende Ausnehmung der anderen Komponente vorschraubbar ist. Bei einer Reoperation ist die Sicherungsschraube nicht ohne weiteres zugänglich. Dadurch wird die Entnahme der auszutauschenden Prothese erschwert. Die oben genannte Druckschritt EP-A-474 015 zeigt alle Merkmale des Oberbegriffs des Anspruchs 1.

Der Erfindung liegt daher die Aufgabe zugrunde, die Lösbarkeit eines derartigen Sicherungsbolzens für den Fall der Reoperation zu erleichtern.

Die erfindungsgemäße Lösung besteht in einer Endoprothese gemäß Anspruch 1. Demgemäß kann die im Anspruch 1 genannte Verbindung dadurch gelöst werden, daß der Sicherungsbolzen gebrochen oder abgeschert wird. Dafür wird eine Belastung von einer Größe aufgebracht, die bei normaler Benutzung der Prothese nicht zu erwarten ist. Um das Brechen oder Abscheren zu erleichtern, wird der Sicherungsbolzen spröde oder weich oder mit einer Sollbruchstelle ausgeführt.

Beispielsweise wird im Falle eines spröden Sicherungsbolzens bei der Reoperation auf eine der beiden Komponenten in Löserichtung mittels eines geeigneten Instruments ein Schlag ausgeübt, durch den der Sicherungszapfen gebrochen wird und durch den in der Regel auch gleichzeitig der Verbindungszapfen in der Verbindungsbohrung gelöst wird, so daß die Komponenten anschließend leicht voneinander getrennt werden können.

In der Regel wird als Sicherungsbolzen ein Schraubbolzen verwendet; dies ist jedoch nicht unbedingt erforderlich.

Damit das Bruchstück des Sicherungsbolzens sich zwischen den beiden Komponenten nicht so verklemmt, daß es ihre Trennung behindert, soll in seiner Nachbarschaft eine Ausnehmung vorgesehen sein, die zur Aufnahme des Bruchstücks ausreichend bemessen ist. Zweckmäßigerweise wird dazu die Ausnehmung verwendet, in die die Teillänge des Bolzens im Sicherungszustand eingreift.

Die Erfindung wird im folgenden näher unter Bezugnahme auf die Zeichnung erläutert, die vorteilhafte Ausführungsbeispiele veranschaulicht. Es zeigen:
- Fig. 1: eine Konusverbindung zwischen der femoralen Komponente einer Kniegelenkendoprothese und dem zugehörigen Markhöhlenzapfen und
- Fig. 2: einen Teilschnitt durch die Anordnung gemäß Fig. 1 in einer Ausführungsvariante.

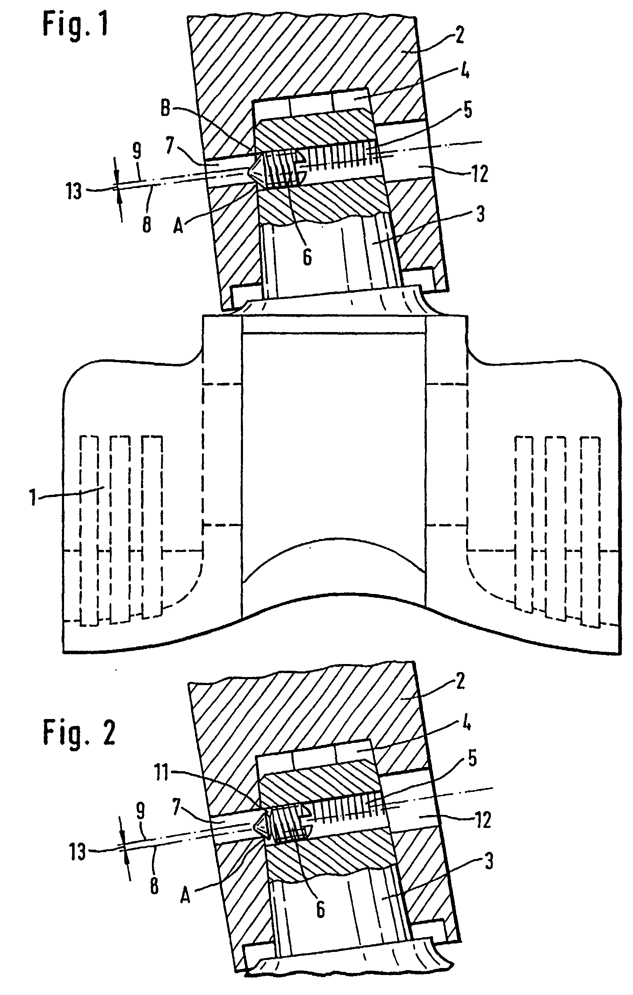

In der Ausführungsform gemäß Fig. 1 ist die femorale Komponente 1 einer Kniegelenkendoprothese mit einem Markhöhlenschaft verbunden, dessen unterer Abschnitt 2 teilweise geschnitten dargestellt ist. Mit der Komponente 1 ist ein Konuszapfen 3 verbunden, der in eine Konusbohrung 4 des Markhöhlenschafts eingesetzt ist. Innerhalb des Konuszapfens ist eine Gewindebohrung 5 vorgesehen, die eine Sicherungsschraube 6 enthält, deren Spitze in eine Bohrung 7 vorschraubbar ist, die als zugehörige Sicherungsausnehmung dient. Die Achsen 8 und 9 der Bohrungen 5 und 7 sind um den Betrag 13 zueinander versetzt, so daß die Spitze der Sicherungsschraube 6 mit derjenigen Kante A der Bohrung 7 zusammenwirkt, die auf der in Löserichtung liegenden Seite der Bohrung 7 liegt. Beim Anziehen der Schraube 6 wird dadurch eine die Konusverbindung 3, 4 verstärkende Kraft erzeugt. Die andere Kante B der Bohrung 7 wird im Normalfall von der Sicherungsschraube nicht erreicht.

Vor der Montage liegt die Sicherungsschraube 6 vollständig in der Gewindebohrung 5, so daß der Konus 3 in die Bohrung 4 eingesetzt werden kann. Die Sicherungsschraube ist dann durch die Bohrung 12 in Teil 2 zugänglich, so daß sie in die Sicherungslage verschraubt werden kann. Gewünschtenfalls kann eine (nicht gezeigte) Konterschraube nachgesetzt werden, um die Sicherungsschraube 6 in der Sicherungslage zu sichern.

Die Sicherungsschraube 6 besteht aus sprödem Material, das aber hinreichend fest ist, um die Sicherungsfunktion während der normalen Benutzung der Prothese zu gewährleisten. Im Falle der Reoperation wird mittels eines geeigneten Werkzeugs ein Schlag auf die Komponente 1 in Löserichtung ausgeübt. Dadurch bricht die Spitze der Sicherungsschraube ab und fällt in die Bohrung 7. Die Teile können dann leicht voneinander entfernt werden.

Alternativ kann die Sicherungsschraube 6 aus einem weichen Werkstoff bestehen, dessen Festigkeit zwar für die Sicherungsfunktion ausreicht, der aber abgeschert wird, wenn beispielsweise mit einem Spreizwerkzeug eine die Komponenten 1 und 2 trennende Kraft ausgeübt wird.

In der Ausführungsalternative der Fig. 2, deren Konstruktionszusammenhang (soweit nicht gezeigt) demjenigen der Fig. 1 gleichen kann, ist die sichernd mit der Bohrung 7 zusammenwirkende Spitze der Sicherungsschraube 6 über eine Sollbruchstelle 11 von dem Hauptteil der Schraube getrennt. Dadurch wird sichergestellt, daß das Bruchstück, das nach dem Abbrechen oder Abscheren der Schraubenspitze sich bildet, nicht zu groß ist, um von der Bohrung 7 aufgenommen zu werden. Falls man mit einer Bruchstückgröße rechnen muß, die das Aufnahmevermögen der Bohrung 7 übersteigt, so kann diese ohne weiteres zur Seite ihrer Kante B hin größer ausgeführt werden, da es für die Sicherungsfunktion lediglich auf die unverändert bleibende Kante A ankommt.

## Patentansprüche

1. Endoprothese, die zur Verbindung von mindestens zwei ihrer Komponenten (1,2) einen Verbindungszapfen (3) an der einen (1) und eine diesen aufnehmende Verbindungsbohrung (4) an der anderen Komponente aufweist, wobei zur gegenseitigen Lagesicherung ein Sicherungsbolzen (6) in einer Bolzenbohrung (5) in einer der beiden Komponenten (1) und eine Ausnehmung (7) zur Aufnahme einer Teillänge des Sicherungsbolzens (6) in der anderen Komponente (2) vorgesehen sind, **dadurch gekennzeichnet, daß** der Sicherungsbolzen eine Sollbruchstelle (11) aufweist.

2. Endoprothese nach Anspruch 1, **dadurch gekennzeichnet, daß** die Ausnehmung (7) eine zur Aufnahme des abgetrennten Stücks des Sicherungsbolzens (6) ausreichende Größe aufweist.

## Claims

1. Endoprosthesis which, for connecting at least two of its components (1, 2), has a connection plug (3) on one component (1) and a connection bore (4) on the other component for receiving this connection plug, in which endoprosthesis, for the purpose of mutual positional securing, a securing bolt (6) is provided within a bolt bore (5) in one of the two components (1), and a recess (7) for receiving part of the length of the securing bolt (6) is provided in the other component (2), **characterized in that** the securing bolt has a predetermined break point (11).

2. Endoprosthesis according to Claim 1, **characterized in that** the recess (7) is of a size which is sufficient to receive the detached section of the securing bolt (6).

## Revendications

1. Endoprothèse qui comporte, pour la liaison d'au moins deux de ses composants (1, 2), un tenon de liaison (3) sur un composant (1) et une mortaise de liaison (4), recevant celui-ci, sur l'autre composant, dans laquelle, pour le blocage réciproque en position, il est prévu un boulon de sûreté (6) dans un trou pour boulon (5) dans l'un des deux composants (1) et un évidement (7) destiné à recevoir une partie de la longueur du boulon de sûreté (6) dans l'autre composant (2), **caractérisée en ce que** le boulon de sûreté présente une zone destinée à la rupture (11).

2. Endoprothèse selon la revendication 1, **caractérisée en ce que** l'évidement (7) présente une taille suffisante pour recevoir la partie sectionnée du boulon de sûreté (6).
